# EUROPEAN PATENT APPLICATION

(11) **EP 4 234 579 A1**
(43) Date of publication of application: **30.08.2023**
(21) Application number: 21882137.9
(22) Date of filing: 22.10.2021
(51) Int. Cl.: C07K 16/28, C12N 15/13

(54) **ANTI-CD73 ANTIBODY AND USE THEREOF**

(30) Priority: 23.10.2020 CN 202011152518
(71) Applicant: Akeso Biopharma, Inc., Zhongshan, Guangdong 528437 (CN); Akeso Pharmaceuticals, Inc., Guangdong 510799 (CN)
(72) Inventor: LI, Baiyong, Zhongshan, Guangdong 528437 (CN); XIA, Yu, Zhongshan, Guangdong 528437 (CN); WANG, Zhongmin, Zhongshan, Guangdong 528437 (CN)
(74) Representative: Altmann Stößel Dick Patentanwälte PartG mbB
(86) International application number: PCT/CN2021/125564
(87) International publication number: WO 2022/083723

(57) **Abstract**

An anti-CD73 antibody and the use thereof. The heavy chain variable region of the antibody comprises HCDR1-HCDR3 having amino acid sequences as shown in SEQ ID NOs: 15-17; and the light chain variable region of the antibody comprises LCDR1-LCDR3 having amino acid sequences as shown in SEQ ID NOs: 18-20.

## Description

### TECHNICAL FIELD

The present invention relates to the field of immunology, and in particular to an anti-CD73 antibody and use thereof.

### BACKGROUND

Ecto-5'-nucleotidase, namely CD73 protein, is a multifunctional glycoprotein encoded by NT5E gene and having a molecular weight of 70 KD, which is anchored on the cell membrane by glyocsyl phosphatidy linositol (GPI) (Zimmermann H., Biochem J., 1992; 285:345-365).

CD73 is widely distributed on the surface of human tissue cells, and it has been found in research that CD73 is highly expressed in various solid tumors, specifically in cancer cells, dendritic cells, regulatory T cells (Tregs), natural killer cells (NK cells), myeloid-derived suppressor cells (MDSCs), tumor-associated macrophages (TAMs) and the like in a tumor microenvironment. Hypoxia induces the up-regulation of molecules such as hypoxia-inducible factor-1 (HIF-1), thereby leading to the widespread expression of CD73 in the tumor microenvironment (Synnestvedt K, et al. J Clin Invest., 2002; 110:993-1002). Analysis of clinical tumor samples has shown that high expression of CD73 is a potential biomarker and is closely related to adverse prognosis of various types of tumors, including breast cancer, lung cancer, ovarian cancer, kidney cancer, gastric cancer, head and neck cancer and the like.

CD73 has both hydrolase activity and non-hydrolase activity. The enzyme and non-enzyme functions of CD73 simultaneously work in the related process in tumors, and mutually promote and maintain the progression of tumors. More and more studies have found that CD73 is a key regulatory molecule for tumor cell proliferation, metastasis and invasion *in vitro,* and tumor angiogenesis and tumor immune escape mechanism *in vivo,* wherein an important immune suppression mechanism is mediated by CD73-adenosine metabolic signaling pathway. CD39 at the upstream of CD73 can catalyze ATP to generate adenosine monophosphate (AMP), the generated AMP is converted into adenosine by CD73, and adenosine binds to a downstream adenosine receptor (A2AR). A2AR inhibits a series of signaling pathways related to immune activation, such as LCK, MAPK, and PKC, and inhibits the immune killing effect of T cells by activating protein kinase A (PKA) and Csk kinase, thereby playing an immune suppression role (Antonioli L, et al. Nat Rev Cancer. 2013; 13:842-857).

The transmembrane receptor PD-1 (programmed cell death protein 1) is a member of the CD28 family, and is expressed in activated T cells, B cells and myeloid cells. Both ligands of PD-1, PDL1 (programmed cell death 1 ligand 1, or PDL-1) and PDL2 (programmed cell death 1 ligand 2, or PDL-2), are members of the B7 superfamily. PDL1 is expressed in a variety of cells including T cells, B cells, endothelial cells and epithelial cells, and PDL2 is expressed only in antigen-presenting cells such as dendritic cells and macrophages.

The PD-1/PDL1 signaling pathway plays an important role in regulating immune tolerance, microbial infection and tumor immune escape. PD-1 is mainly expressed in immune cells such as T cells, and the ligand PDL1 of PD-1 is highly expressed in a plurality of human tumor tissues. Blocking the PD-1/PDL1 signaling pathway may activate inhibited T cells, which thus attack cancer cells. Blocking the PD-1/PDL1 signaling can promote the proliferation of tumor antigen-specific T cells, activate tumor cell killing process and further inhibit local tumor growth (Julie R et al., 2012, A Engl J Med., 366:2455-2465). In addition, tumors with high PDL1 expression are associated with cancers that are difficult to detect (Hamanishi et al., 2007, Proc. Natl. Acad. Sci. USA, 104:3360-5). An effective method is administering an anti-PD-1 antibody to modulate the expression of PD-1. Due to the broad anti-tumor prospects and surprising efficacy of PD-1 antibodies, it is widely accepted in the industry that antibodies targeting the PD-1 pathway will bring about breakthroughs in various tumors, for example, non-small cell lung cancer, renal cell carcinoma, ovarian cancer, melanoma (Hornet M. B., Parisi G., et al., 2015, Semin Oncol., 42(3):466-473), leukemia and anemia (Held SA, Heine A, et al., 2013, Curr Cancer Drug Targets., 13(7):768-74).

Cytotoxic T lymphocyte-associated antigen 4 (CTLA4) and CD28 molecules are very similar in aspects of gene structure, chromosome location, sequence homology and gene expression. Both molecules are receptors of co-stimulatory molecule B7, and mainly expressed on the surface of activated T cells. Binding of CTLA4 to B7 inhibits the activation of mouse and human T cells, and plays a negative regulatory role in T cell activation.

CTLA4 antibodies (or anti-CTLA4 monoclonal antibodies) or CTLA4 ligands can prevent CTLA4 from binding to its natural ligands, thereby blocking the transmission of negative regulatory signals by CTLA4 to T cells and enhancing the reactivity of T cells to various antigens. In this respect, *in-vivo* and *in-vitro* studies are essentially consistent. Currently, there are CTLA4 monoclonal antibodies in clinical trials or approved for treating prostate cancer, bladder cancer, colorectal cancer, gastrointestinal cancer, liver cancer, malignant melanoma, etc. (Grosso JF., Jure-Kunkel MN., 2013, Cancer Immun., 13:5).

CTLA4 and CTLA4 antibodies are important influencing factors of T cell functions and play a role by interfering with the immune microenvironment in the body. *In-vitro* and *in-vivo* studies demonstrated that CTLA4 antibodies can specifically relieve the immunosuppression of CTLA4, activate T cells, and induce IL-2 generation, and are promising in wide applications in gene therapy against diseases such as tumors and parasite infections. CTLA4 antibodies can produce specific therapeutic effect on diseases and show remarkable efficacy, and may be used for supplementing traditional medicines and for exploring new means of gene therapy.

ADCC (antibody-dependent cell-mediated cytotoxicity) refers to direct killing of a target cell by a killer cell (NK cell, macrophage, etc.) that is mediated by binding of the Fab fragment of an antibody to an epitope of a virus-infected cell or a tumor cell and binding of the Fc fragment of the antibody to an Fc receptor (FcR) on the surface of the killer cell.

CDC (complement dependent cytotoxicity) means that the specific binding of an antibody to a corresponding antigen on a cell membrane surface forms a complex and activates the complement system, which further forms an MAC on the surface of the target cell resulting in subsequent target cell lysis. Complements may cause lysis of various bacteria and other pathogenic organisms, and are an important defense mechanism against pathogenic organism infections.

Fc receptors belong to an immunoglobulin family that are expressed on the surface of specific immune cells to recognize antibody Fc regions and mediate immune responses. After the Fab region recognizes an antigen, the Fc region of the antibody binds to the Fc receptor on the immune cell (e.g., a killer cell) to initiate the response function of the immune cell, such as phagocytosis and ADCC. According to the type of antibody recognized by the Fc receptor and the type of expression cells, FcγRIIIa is found to be closely associated with ADCC effect. FcγRIIIa is the most predominant molecule mediating ADCC (Hogarth PM, Pietersz GA., 2012, NATURE REVIEWS DRUG DISCOVERY, 11(4):311-331).

The IgG family comprises four members, IgG1, IgG2, IgG3 and IgG4, which differ in amino acids in the fragment crystallizable (Fc) region of the heavy chain constant region, resulting in their varying affinities for FcγRs. IgG1 is the most abundant subtype in humans and is also the most common subtype used in monoclonal antibody medication. IgG1 is capable of binding various FcγRs and is able to induce ADCC and CDC effects. The presence of ADCC and/or CDC in antibodies may cause undesired targeted tissue damage, posing pharmacological adverse effects.

### SUMMARY

After intensive studies and creative efforts, the inventors used mammalian cell expression systems to express recombinant human CD73 as an antigen to immunize mice, and obtained hybridoma cells by fusion of mouse spleen cells and myeloma cells. The inventors obtained a hybridoma cell line LT014 (deposited under CCTCC NO. C2018137) by screening a large number of samples.

The inventors have surprisingly found that the hybridoma cell line LT014 can secrete a specific monoclonal antibody (designated 19F3) specifically binding to human CD73, and the monoclonal antibody can effectively inhibit the enzyme activity reaction of CD73 in a non-substrate competition mode, reduce the production of adenosine, promote the activity of T cells, and exert the effect of inhibiting tumor growth.

Further, the inventors have creatively prepared humanized antibodies against human CD73 (designated 19F3H1L1(hG1DM), 19F3H2L2(hG1DM), 19F3H2L3 and 19F3H2L3(hG1DM)), and further, the anti-CD73 antibodies contain amino acid mutations to eliminate ADCC and CDC effects, avoiding undesired antibody-mediated toxicity.

The inventors have also surprisingly found that the combination of the antibody of the present invention and an anti-PD-1/CTLA-4 bispecific antibody has a pharmacological effect of effectively inhibiting tumor cell proliferation, which is superior to that of either the anti-PD-1/CTLA-4 bispecific antibody or the anti-CD73 antibody alone.

Another aspect of the present invention further relates to an antibody, wherein the anti-CD73 antibody comprises:
HCDR1, HCDR2 and HCDR3 of a heavy chain variable region set forth in SEQ ID NO: 2, SEQ ID NO: 6 or SEQ ID NO: 10; and LCDR1, LCDR2 and LCDR3 of a light chain variable region set forth in SEQ ID NO: 4, SEQ ID NO: 8, SEQ ID NO: 12 or SEQ ID NO: 14;
preferably, according to an IMGT numbering system, the anti-CD73 antibody comprises:
   HCDR1 comprising or consisting of an amino acid sequence set forth in SEQ ID NO: 15, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
   HCDR2 comprising or consisting of an amino acid sequence set forth in SEQ ID NO: 16, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
   HCDR3 comprising or consisting of an amino acid sequence set forth in SEQ ID NO: 17, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
   LCDR1 comprising or consisting of an amino acid sequence set forth in SEQ ID NO: 18, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
   LCDR2 comprising or consisting of an amino acid sequence set forth in SEQ ID NO: 19, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence, and
   LCDR3 comprising or consisting of an amino acid sequence set forth in SEQ ID NO: 20, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence.

In some embodiments of the present invention,
a heavy chain variable region of the antibody comprises or consists of the following sequences:
   SEQ ID NO: 2, SEQ ID NO: 6 or SEQ ID NO: 10, a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 2, SEQ ID NO: 6 or SEQ ID NO: 10, or an amino acid sequence having one or more (preferably 1, 2, 3, 4, 5, 6, 7, 8, 9 or 10) conservative amino acid mutations (preferably substitutions, insertions or deletions) compared with the amino acid sequence set forth in SEQ ID NO: 2, SEQ ID NO: 6 or SEQ ID NO: 10; and
a light chain variable region of the antibody comprises or consists of the following sequences:
   SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity to SEQ ID NO: 4, SEQ ID NO: 8, SEQ ID NO: 12 or SEQ ID NO: 14, or an amino acid sequence having one or more (preferably 1, 2, 3, 4, 5, 6, 7, 8, 9 or 10) conservative amino acid mutations (preferably substitutions, insertions or deletions) compared with the amino acid sequence set forth in SEQ ID NO: 4, SEQ ID NO: 8, SEQ ID NO: 12 or SEQ ID NO: 14.

In some embodiments of the present invention, the heavy chain variable region of the antibody comprises an amino acid sequence set forth in SEQ ID NO: 2, and the light chain variable region of the antibody comprises an amino acid sequence set forth in SEQ ID NO: 4;
the heavy chain variable region of the antibody comprises an amino acid sequence set forth in SEQ ID NO: 6, and the light chain variable region of the antibody comprises an amino acid sequence set forth in SEQ ID NO: 8;
the heavy chain variable region of the antibody comprises an amino acid sequence set forth in SEQ ID NO: 10, and the light chain variable region of the antibody comprises an amino acid sequence set forth in SEQ ID NO: 12; or the heavy chain variable region of the antibody comprises an amino acid sequence set forth in SEQ ID NO: 10, and the light chain variable region of the antibody comprises an amino acid sequence set forth in SEQ ID NO: 14.

In some embodiments of the present invention, a heavy chain constant region of the antibody is an Ig gamma-1 chain C region, ACCESSION: P01857; a light chain constant region is an Ig kappa chain C region, ACCESSION: P01834.

In some embodiments of the present invention, the heavy chain constant region of the antibody is an Ig gamma-1 chain C region, ACCESSION: P01857, having a leucine-to-alanine point mutation at position 234 (L234A), and a leucine-to-alanine point mutation at position 235 (L235A); the light chain constant region is an Ig kappa chain C region, ACCESSION: P01834, having an amino acid sequence set forth in SEQ ID NO: 22.

The variable regions of the light chain and the heavy chain determine antigen binding; the variable region of each chain contains three hypervariable regions called complementarity determining regions (CDRs) (CDRs of the heavy chain (H) include HCDR1, HCDR2 and HCDR3, and CDRs of the light chain (L) include LCDR1, LCDR2 and LCDR3, which are named by Kabat et al., see Bethesda M.d., Sequences of Proteins of Immunological Interest, Fifth Edition, NIH Publication 1991; 1-3:91-3242).

Preferably, CDRs may also be defined by the IMGT numbering system, see Ehrenmann, Francois, Quentin Kaas, and Marie-Paule Lefranc. IMGT/3Dstructure-DB and IMGT/DomainGapAlign: a database and a tool for immunoglobulins or antibodies, T cell receptors, MHC, IgSF and MhcSF. Nucleic acids research 2009; 38(suppl_1): D301-D307.

The amino acid sequences of the CDRs of the monoclonal antibody sequences are analyzed according to the IMGT definition by technical means well known to those skilled in the art, for example by using the VBASE2 database.

The antibodies 19F3, 19F3H1L1(hG1DM), 19F3H2L2(hG1DM) and 19F3H2L3(hG1DM) involved in the present invention have the same CDRs: the 3 CDRs of their heavy chain variable regions have the following amino acid sequences:
HCDR1: GYSFTGYT (SEQ ID NO: 15),
HCDR2: INPYNAGT (SEQ ID NO: 16), and
HCDR3: ARSEYRYGGDYFDY (SEQ ID NO: 17);
the 3 CDRs of their light chain variable regions have the following amino acid sequences:
LCDR1: QSLLNSSNQKNY (SEQ ID NO: 18),
LCDR2: FAS (SEQ ID NO: 19), and
LCDR3: QQHYDTPYT (SEQ ID NO: 20).

In some embodiments of the present invention, the antibody is a monoclonal antibody.

In some embodiments of the present invention, the antibody is a humanized antibody, a chimeric antibody or a multispecific antibody (e.g., a bispecific antibody).

In some embodiments of the present invention, the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody.

Another aspect of the present invention relates to an isolated polypeptide selected from the group consisting of:
(1) an isolated polypeptide, comprising sequences set forth in SEQ ID NO: 15, SEQ ID NO: 16 and SEQ ID NO: 17, wherein the polypeptide, as part of an anti-CD73 antibody, specifically binds to CD73, the antibody further comprising sequences set forth in SEQ ID NO: 18, SEQ ID NO: 19 and SEQ ID NO: 20;
(2) an isolated polypeptide, comprising sequences set forth in SEQ ID NO: 18, SEQ ID NO: 19 and SEQ ID NO: 20, wherein the polypeptide, as part of an anti-CD73 antibody, specifically binds to CD73, the antibody further comprising sequences set forth in SEQ ID NO: 15, SEQ ID NO: 16 and SEQ ID NO: 17;
(3) an isolated polypeptide, comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence;
(4) an isolated polypeptide, comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence;
(5) an isolated polypeptide, comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence; and
(6) an isolated polypeptide, comprising a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence.

Another aspect of the present invention relates to an isolated nucleic acid molecule encoding the antibody or the antigen-binding fragment thereof or the isolated polypeptide according to any one of the embodiments of the present invention.

Yet another aspect of the present invention relates to a vector comprising the isolated nucleic acid molecule of the present invention.

Yet another aspect of the present invention relates to a host cell comprising the isolated nucleic acid molecule of the present invention or the vector of the present invention.

Yet another aspect of the present invention relates to a conjugate comprising an antibody and a conjugated moiety, wherein the antibody is the antibody or the antigen-binding fragment thereof according to any one of the embodiments of the present invention, and the conjugated moiety is a purification tag (e.g., a His tag) or a detectable label; preferably, the conjugated moiety is a radioisotope, a fluorescent substance, a chemiluminescent substance, a colored substance, polyethylene glycol or an enzyme.

Yet another aspect of the present invention relates to a fusion protein or a multispecific antibody (preferably a bispecific antibody) comprising the antibody or the antigen-binding fragment thereof according to any one of the embodiments of the present invention.

Yet another aspect of the present invention relates to a kit comprising the antibody or the antigen-binding fragment thereof according to any one of the embodiments of the present invention, the conjugate, the fusion protein or the multispecific antibody of the present invention; preferably, the kit further comprises a second antibody specifically recognizing the antibody; optionally, the second antibody further comprises a detectable label, such as a radioisotope, a fluorescent substance, a chemiluminescent substance, a colored substance or an enzyme.

Yet another aspect of the present invention relates to use of the antibody or the antigen-binding fragment thereof according to any one of the embodiments of the present invention, the conjugate, the fusion protein or the multispecific antibody of the present invention in preparing a kit for detecting the presence or level of CD73 in a sample.

Yet another aspect of the present invention relates to a pharmaceutical composition comprising the antibody or the antigen-binding fragment thereof according to any one of the embodiments of the present invention, the conjugate, the fusion protein or the multispecific antibody of the present invention; optionally, the pharmaceutical composition further comprises a pharmaceutically acceptable carrier and/or excipient. Preferably, the pharmaceutical composition is in a form suitable for administration by subcutaneous injection, intradermal injection, intravenous injection, intramuscular injection or intralesional injection.

Yet another aspect of the present invention relates to use of the antibody or the antigen-binding fragment thereof according to any one of the embodiments of the present invention, the conjugate, the fusion protein or the multispecific antibody of the present invention in preparing a medicament for treating and/or preventing a tumor (such as a solid tumor, preferably non-small cell lung cancer, prostate cancer (including metastatic castration-resistant prostate cancer (mCRPC)), triple-negative breast cancer, ovarian cancer, colorectal cancer (including microsatellite stability (MSS) and mismatch repair dysfunction/microsatellite high instability (dMMR/MSI-high) type), gastric cancer (including microsatellite stability (MSS) and mismatch repair dysfunction/microsatellite high instability (dMMR/MSI-high) type), melanoma, head and neck cancer, renal cell carcinoma or pancreatic ductal adenocarcinoma), or in preparing a medicament for diagnosing a tumor.

Yet another aspect of the present invention relates to a hybridoma cell line LT014, which was deposited at China Center for Type Culture Collection (CCTCC) under CCTCC NO. C2018137.

Yet another aspect of the present invention relates to a kit comprising (1) the antibody or the antigen-binding fragment thereof described herein, the conjugate described herein, or the fusion protein or the multispecific antibody described herein, and (2) an anti-PD-1/CTLA-4 bispecific antibody, and optionally, instructions for use.

Yet another aspect of the present invention relates to a method for treating and/or preventing a tumor, comprising administering to a patient a therapeutically effective amount of drug A and a therapeutically effective amount of drug B, wherein drug A comprises the antibody or the antigen-binding fragment thereof described herein, the conjugate described herein, or the fusion protein or the multispecific antibody described herein, and drug B comprises an anti-PD-1/CTLA-4 bispecific antibody; preferably, drug A and drug B are administered either simultaneously or sequentially, wherein the sequential administration is that drug A is administrated firstly or drug B is administrated firstly.

In some embodiments of the present invention, the anti-PD-1/CTLA-4 bispecific antibody comprises a heavy chain amino acid sequence set forth in SEQ ID NO: 35 and a light chain amino acid sequence set forth in SEQ ID NO: 36.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (V_{H}) and a heavy chain constant region (C_{H}). The heavy chain constant region consists of 3 domains (C_{H1}, C_{H2}, and C_{H3}). Each light chain consists of a light chain variable region (V_{L}) and a light chain constant region (C_{L}). The light chain constant region consists of one domain C_{L}. The constant region of the antibody can mediate the binding of immunoglobulins to host tissues or factors, including the binding of various cells of the immune system (e.g., effector cells) to the first component (C1q) of classical complement system. The V_{H} and V_{L} regions can be further subdivided into hypervariable regions (called complementarity determining regions, or CDRs) and conservative regions called framework regions (FRs) that are distributed between the CDRs. Each V_{H} and V_{L} consists of 3 CDRs and 4 FRs arranged from the amino terminus to the carboxyl terminus in the following order: FR1, CDR1, FR2, CDR2, FR3, CDR3, FR4. The variable regions (V_{H} and V_{L}) of each heavy chain/light chain pair form antigen-binding sites, respectively. The assignment of amino acids to the regions or domains is based on Kabat Sequences of Proteins of Immunological Interest (National Institutes of Health, Bethesda, M.d. (1987 and 1991)), or Chothia & Lesk J. Mol. Biol. 1987; 196:901-917; Chothia et al. Nature 1989; 342:878-883 or the definition of the IMGT numbering system, see the definition in Ehrenmann, Francois, Quentin Kaas, and Marie-Paule Lefranc. "IMGT/3Dstructure-DB and IMGT/DomainGapAlign: a database and a tool for immunoglobulins or antibodies, T cell receptors, MHC, IgSF and MhcSF." Nucleic acids research 2009; 38(suppl_1): D301-D307. The term "antibody" is not limited by any specific method for producing the antibody. For example, the antibody includes, in particular, a recombinant antibody, a monoclonal antibody and a polyclonal antibody. The antibody can be antibodies of different isotypes, such as IgG (e.g., subtypes IgG1, IgG2, IgG3 or IgG4), IgA1, IgA2, IgD, IgE or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or antibody fragment obtained when all or a part of CDRs of a human immunoglobulin (receptor antibody) are replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature 1986; 321:522-525; Reichmann et al., Nature 1988; 332:323-329; Presta, Curr. Op. Struct. Biol., 1992; 2:593-596; and Clark M. Antibody humanization: a case of the 'Emperor's new clothes'? [J]. Immunol. Today, 2000; 21(8): 397-402.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements and reporter genes. In addition, the vector may further comprise a replication initiation site.

As used herein, the term "host cell" refers to cells to which vectors can be introduced, including, but not limited to, prokaryotic cells such as *E*. *coli* or *bacillus subtilis,* fungal cells such as yeast cells or *aspergillus*, insect cells such as S2 drosophila cells or Sf9, or animal cells such as fibroblasts, CHO cells, COS cells, NSO cells, HeLa cells, GS cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, e.g., less than about 10⁻⁶ M, 10⁻⁷ M, 10⁻⁸ M, 10⁻⁹ M or 10⁻¹⁰ M or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, e.g., less than about 10⁻⁶ M, 10⁻⁷ M, 10⁻⁸ M, 10⁻⁹ M or 10⁻¹⁰ M or less. K_{D} can be determined using methods known to those skilled in the art, e.g., using a Fortebio system.

As used herein, the terms "monoclonal antibody" and "mAb" have the same meaning and are used interchangeably; the terms "polyclonal antibody" and "pAb" have the same meaning and are used interchangeably; the terms "polypeptide" and "protein" have the same meaning and are used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop diseases and complications thereof in patients suffering from the disease.

### Beneficial effects of the present invention:

The monoclonal antibody of the present invention can specifically bind to CD73 well, and can effectively inhibit the enzyme activity reaction of CD73 in a non-substrate competition mode, reduce the production of adenosine, and promote the activity of T cells and the tumor inhibitory effect. Meanwhile, the combination of the antibody of the present invention and an anti-PD-1/CTLA-4 bispecific antibody has a pharmacological effect of effectively inhibiting tumor cell proliferation, which is superior to that of either the anti-PD-1/CTLA-4 bispecific antibody or the anti-CD73 antibody alone.

### BRIEF DESCRIPTION OF THE DRAWINGS

FIG. 1 shows the fitting curve of dynamic affinity data of 19F3H2L3(hG1DM) binding to C1q.
FIG. 2 shows the fitting curve of dynamic affinity data of MEDI9447 binding to C1q.
FIG. 3 shows the fitting curve of dynamic affinity data of wild-type IgG1 antibody binding to C1q.
FIG. 4 shows the fitting curve of dynamic affinity data of 19F3H2L3(hG1DM) binding to FcγRIIIa.
FIG. 5 shows the fitting curve of dynamic affinity data of MEDI9447 binding to FcγRIIIa.
FIG. 6 shows the fitting curve of dynamic affinity data of wild-type IgG1 antibody binding to FcγRIIIa.
FIG. 7 shows tumor weights of each group of mice on Day 23 after grouping. ** P < 0.01.
FIG. 8 shows rates of change in the body weight of each group of animals during the experiment compared with Day 0.
FIG. 9 shows the anti-CD73 antibody effectively inhibiting CD73 enzymatic activity.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the product manual. Reagents or instruments used are commercially available conventional products if the manufacturers thereof are not specified.

In the following examples of the present invention, BALB/c mice used were purchased from Guangdong Medical Laboratory Animal Center.

In the following examples of the present invention, the positive control antibody MEDI9447 (Oleclumab) used was produced by Akeso Biopharma, Inc., the sequence of which was identical to the antibody sequence described in International Nonproprietary Names for Pharmaceutical Substances (INN) publicly published by Medlmmune Limited at the WHO site (World Health Organization (2016). "International Nonproprietary Names for Pharmaceutical Substances (INN). Proposed INN: List 116" (PDF). WHO Drug Information. 30(4), P661-662).

In the following examples of the present invention, the heavy chain constant region of the positive control antibody wild-type IgG1 control antibody used was Ig gamma-1 chain C region, ACCESSION: P01857; the light chain constant region was Ig kappa chain C region, ACCESSION: P01834.

In the following examples of the present invention, C1q used was purchased from fizgerald, Cat No. A16050201;

in the following examples of the present invention, FcγRIIIa-bio used was purchased from Sino Biological, Cat No. LC09JA0407;

in the following examples of the present invention, the CD73 (5'-nuclease) specific inhibitor APCP (alpha, beta-methylene adenosine-5'-diphosphate, 5'-α, β-methylene-adenosine diphosphate) used was derived from Sigma, Cat No. M3763-10MG.

In the following examples of the present invention, the sequence of the isotype control antibody, human anti-hen egg lysozyme IgG (i.e., anti-HEL antibody, or human IgG, abbreviated as hIgG, or isotype control) was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1):130-146).

### Example 1: Preparation of Anti-CD73 Antibody 19F3

### 1. Preparation of hybridoma cell line LT014

The antigen used to prepare the anti-CD73 antibody was human NT5E-His (for NT5E, GenbankID: NP_002517.1, position: 1-552, prepared by Akeso Biopharma, Inc.). Spleen cells of immunized mice were fused with myeloma cells of the mice to prepare hybridoma cells. Hybridoma cells were screened by indirect ELISA using human NT5E-Biotin (for NT5E, GenbankID: NP_002517.1, position: 1-552, prepared by Akeso Biopharma, Inc.) as an antigen, and hybridoma cells capable of secreting an antibody specifically binding to CD73 were obtained. The hybridoma cells obtained by screening were subjected to limiting dilution to obtain a stable hybridoma cell line. The hybridoma cell line was designated hybridoma cell line LT014, and the monoclonal antibody secreted therefrom was designated 19F3.

The hybridoma cell line LT014 (also called CD73-19F3) was deposited at China Center for Type Culture Collection (CCTCC) on June 21, 2018 under CCTCC NO. C2018137, the depository address being Wuhan University, Wuhan, China, postal code: 430072.

### 2. Preparation of anti-CD73 antibody 19F3

The LT014 cell line prepared above was cultured with a chemical defined medium (CD medium, containing 1% penicillin-streptomycin) in a 5% CO₂ cell incubator at 37 °C. After 7 days, the cell culture supernatant was collected, subjected to high-speed centrifugation and vacuum filtration through a microfiltration membrane, and purified by using a HiTrap protein A HP column to obtain an antibody 19F3.

### Example 2: Sequence Analysis of Anti-CD73 Antibody 19F3

mRNA was extracted from the cell line LT014 cultured in Example 1 according to the method described in the manual of RNAprep pure Cell/Bacteria Kit (Tiangen, Cat. No. DP430).

cDNA was synthesized according to the manual of Invitrogen SuperScript^{®} III First-Strand Synthesis System for RT-PCR and amplified by PCR.

The PCR amplification products were directly subjected to TA cloning according to the manual of the pEASY-T1 Cloning Kit (Transgen CT101). The TA cloning products were directly sequenced, and the sequencing results of the anti-CD73 antibody 19F3 were as follows:
The nucleotide sequence of the heavy chain variable region is set forth in SEQ ID NO: 1 with a length of 363 bp.

The encoded amino acid sequence is set forth in SEQ ID NO: 2 with a length of 121 aa;
wherein the sequences of heavy chain CDR1, CDR2 and CDR3 are set forth in SEQ ID NOs: 15, 16 and 17, respectively.

The nucleotide sequence of the light chain variable region is set forth in SEQ ID NO: 3 with a length of 339 bp.

The encoded amino acid sequence is set forth in SEQ ID NO: 4 with a length of 113 aa;
wherein the sequences of light chains CDR1, CDR2 and CDR3 are set forth in SEQ ID NOs: 18, 19 and 20, respectively.

### Example 3. Design and Preparation of Light and Heavy Chains of Humanized Anti-Human CD73 Antibodies

The variable region sequences of 19F3H1L1(hG1DM), 19F3H2L2(hG1DM) and 19F3H2L3(hG1DM) were obtained by computer modeling antibody models according to model design mutations based on the sequences of the antibody 19F3 obtained in Example 2 and based on the three-dimensional crystal structure of human CD73 protein (Hage T, Reinemer P, Sebald W., Crystals of a 1:1 complex between human interleukin-4 and the extracellular domain of its receptor alpha chain. Eur JBiochem. 1998; 258(2):831-6); the designed variable region sequences of the humanized antibodies were as follows:
(1) Heavy and light chain variable region sequences of humanized monoclonal antibody 19F3H1L1(hG1DM)
   The nucleotide sequence of the heavy chain variable region is set forth in SEQ ID NO: 5 with a length of 363 bp.
   The encoded amino acid sequence is set forth in SEQ ID NO: 6 with a length of 121 aa, and the sequences of heavy chain CDR1, CDR2 and CDR3 are set forth in SEQ ID NOs: 15, 16 and 17, respectively.
   The nucleotide sequence of the light chain variable region is set forth in SEQ ID NO: 7 with a length of 339 bp.
   The encoded amino acid sequence is set forth in SEQ ID NO: 8 with a length of 113 aa, and the sequences of light chain CDR1, CDR2 and CDR3 are set forth in SEQ ID NOs: 18, 19 and 20, respectively.
(2) Heavy and light chain variable region sequences of humanized monoclonal antibody 19F3H2L2(hG1DM)
   The nucleotide sequence of the heavy chain variable region is set forth in SEQ ID NO: 9 with a length of 363 bp.
   The encoded amino acid sequence is set forth in SEQ ID NO: 10 with a length of 121 aa, and the sequences of heavy chain CDR1, CDR2 and CDR3 are set forth in SEQ ID NOs: 15, 16 and 17, respectively.
   The nucleotide sequence of the light chain variable region is set forth in SEQ ID NO: 11 with a length of 339 bp.
   The encoded amino acid sequence is set forth in SEQ ID NO: 12 with a length of 113 aa, and the sequences of light chain CDR1, CDR2 and CDR3 are set forth in SEQ ID NOs: 18, 19 and 20, respectively.
(3) Heavy and light chain variable region sequences of humanized monoclonal antibody 19F3H2L3(hGlDM)
   The nucleotide sequence of the heavy chain variable region is set forth in SEQ ID NO: 9 with a length of 363 bp.
   The encoded amino acid sequence is set forth in SEQ ID NO: 10 with a length of 121 aa, and the sequences of heavy chain CDR1, CDR2 and CDR3 are set forth in SEQ ID NOs: 15, 16 and 17, respectively.
   The nucleotide sequence of the light chain variable region is set forth in SEQ ID NO: 13 with a length of 339 bp.
   The encoded amino acid sequence is set forth in SEQ ID NO: 14 with a length of 113 aa, and the sequences of light chain CDR1, CDR2 and CDR3 are set forth in SEQ ID NOs: 18, 19 and 20, respectively.

### 3. Preparation of humanized 19F3H1L1(hG1DM), 19F3H2L2(hG1DM) and 19F3H2L3(hGlDM)

The light chain constant regions of 19F3H1L1(hG1DM), 19F3H2L2(hG1DM) and 19F3H2L3(hG1DM) were the Ig kappa chain C region, ACCESSION: P01834.

On the basis of Ig gamma-1 chain C region, ACCESSION: P01857, humanized antibodies were obtained by introducing a leucine-to-alanine point mutation at position 234 (L234A) and a leucine-to-alanine point mutation at position 235 (L235A) in the heavy chain constant region (SEQ ID NO: 21), and were designated 19F3H1L1(hG1DM), 19F3H2L2(hG1DM) and 19F3H2L3(hGlDM).

The heavy chain cDNA and light chain cDNA of 19F3H1L1(hG1DM), the heavy chain cDNA and light chain cDNA of 19F3H2L2(hG1DM) and the heavy chain cDNA and light chain cDNA of 19F3H2L3(hG1DM) were separately cloned into pUC57simple (provided by Genscript) vectors to obtain pUC57simple-19F3H1(hG1DM) and pUC57simple-19F3L1; pUC57simple-19F3H2(hG1DM), pUC57simple-19F3L2 and pUC57simple-19F3L3. With reference to the standard techniques described in Molecular Cloning: A Laboratory Manual (Second Edition), the heavy and light chain full-length genes synthesized by EcoRI&HindIII digestion were subcloned into an expression vector pcDNA3.1 by digestion with a restriction enzyme (EcoRI&HindIII) to obtain expression plasmids pcDNA3.1 -19F3H1 (hGIDM), pcDNA3.1-19F3Ll, pcDNA3.1-19F3H2(hG1DM), pcDNA3.1-19F3L2 and pcDNA3.1-19F3L3, and the heavy/light chain genes of the recombinant expression plasmids were further sequenced. Then the designed gene combinations comprising the corresponding light and heavy chain recombinant plasmids (pcDNA3.1-19F3H1(hG1DM)/pcDNA3.1-19F3L1, pcDNA3.1-19F3H2(hG1DM)/pcDNA3.1-19F3L2, and pcDNA3.1-19F3H2(hG1DM)/pcDNA3.1-19F3L3) were separately co-transfected into 293F cells, and the culture solutions were collected and purified. After the sequences were verified, endotoxin-free expression plasmids were prepared, and were transiently transfected into HEK293 cells for antibody expression. After 7 days of culture, the cell cultures were collected, and subjected to affinity purification on a Protein A column to obtain humanized antibodies.

### Example 4: Dynamic Affinity Assay of Anti-CD73 Antibodies with C1q and FcyRIIIa

### (1) Dynamic affinity assay of anti-CD73 antibodies with C1q

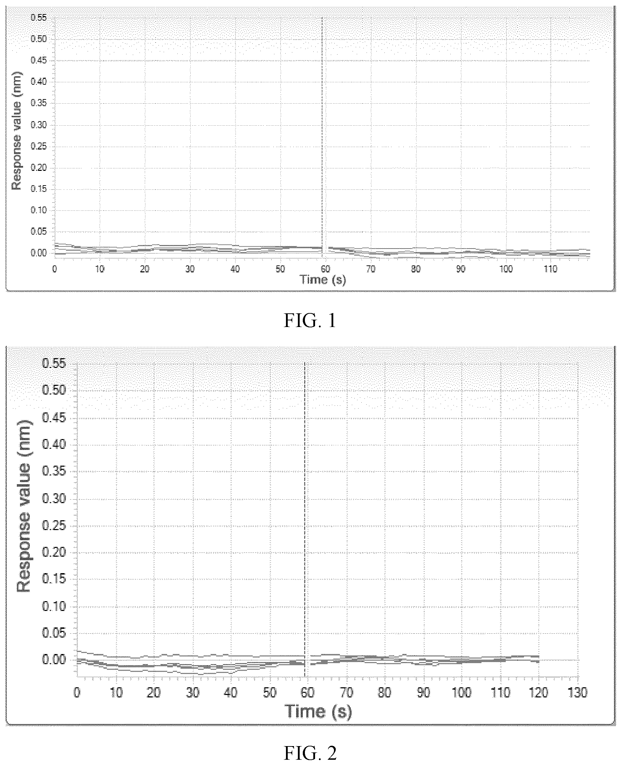

The sample dilution buffer was PBS (0.02% Tween-20, 0.1% BSA, pH 7.4). 50 µg/mL antibody was immobilized on an FAB2G sensor at an immobilization height of about 2.0 nm, and the sensor was equilibrated in a buffer for 60 s. The antibody immobilized on the sensor was allowed to bind to antigen C1q at concentrations of 0.63-10 nM (two-fold serial dilutions) for 60 s, and the antigen-antibody was dissociated in the buffer for 60 s. The sensor was regenerated 4 times with 10 mM glycine pH 1.7, each for 5 s. The sample plate shaking rate was 1000 rpm, the detection temperature was 30 °C and the detection frequency was 0.6 Hz. The data were analyzed by 1:1 model fitting to obtain affinity constants. The data acquisition software was Fortebio Data Acquisition 7.0, and the data analysis software was Fortebio Data Analysis 7.0. According to the results shown in Table 1 and FIGs. 1-3, neither 19F3H2L3(hG1DM) nor MEDI9447 had binding activity to C1q.

| **Sample ID** | **KD (M)** | **kon (1/Ms)** | **Standard error (kon)** | **kdis (1/s)** | **Standard error (kdis)** |
|---|---|---|---|---|---|
| 19F3H2L3(hG1DM) | N/A | N/A | N/A | N/A | N/A |
| MEDI9447 | N/A | N/A | N/A | N/A | N/A |
| Wild-type IgG1 antibody | 1.37E-09 | 5.53E+06 | 4.27E+05 | 7.58E-03 | 4.84E-04 |

| | | | | | |
|---|---|---|---|---|---|
| KD = kdis / kon | | | | | |

### (2) Dynamic affinity assay of anti-CD73 antibodies with FcγRIIIa

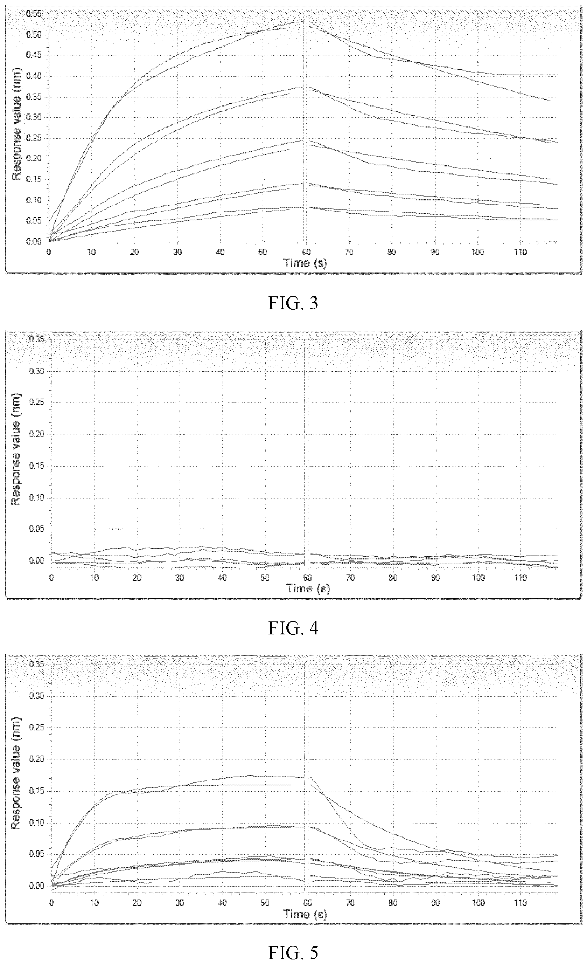

The sample dilution buffer was PBS (0.02% Tween-20, 0.1% BSA, pH 7.4). 0.5 µg/mL FcγRIIIa (from Sino Biological) was immobilized on an SA sensor for 120 s, and the sensor was equilibrated in a buffer for 60 s. The CD16a immobilized on the sensor was allowed to bind to the antibodies at concentrations of 31.3-500 nM (two-fold serial dilutions) for 60 s, and the antibody-antigen was dissociated in the buffer for 60 s. The sensor was regenerated with 10 mM NaOH. The detection temperature was 30 °C and the frequency was 0.6 Hz. The data were analyzed by 1:1 model fitting to obtain affinity constants. According to the results shown in Table 2 and FIGs. 4-6, 19F3H2L3(hG1DM) did not bind to FcyRIIIa, whereas MEDI9447 had binding activity to FcγRIIIa.

| **Sample ID** | **KD (M)** | **kon (1/Ms)** | **Standard error (kon)** | **kdis (1/s)** | **Standard error (kdis)** |
|---|---|---|---|---|---|
| 19F3H2L3(hG1DM) | N/A | N/A | N/A | N/A | N/A |
| MEDI9447 | 1.41E-07 | 2.43E+05 | 4.36E+04 | 3.42E-02 | 2.08E-03 |
| Wild-type IgG1 antibody | 1.25E-07 | 1.76E+05 | 2.22E+04 | 2.20E-02 | 1.11E-03 |

| | | | | | |
|---|---|---|---|---|---|
| KD = kdis / kon | | | | | |

### Example 5: Combination of Anti-CD73 Antibody and Anti-CTLA-4/PD-1 Bispecific Antibody Effectively Blocks Tumor Cell Growth

This study investigated the pharmacological activity of the combination of an anti-CD73 antibody (i.e., 19F3H2L3(hG1DM)) and an anti-PD-1/CTLA-4 bispecific antibody BiAb004 (hG1TM) (the amino acid sequence of the heavy chain is set forth in SEQ ID NO: 35, the amino acid sequence of the light chain is set forth in SEQ ID NO: 36, and the amino acid sequences of the light and heavy chain CDRs are set forth in SEQ ID NO: 23 to SEQ ID NO: 34) in inhibiting tumor growth.

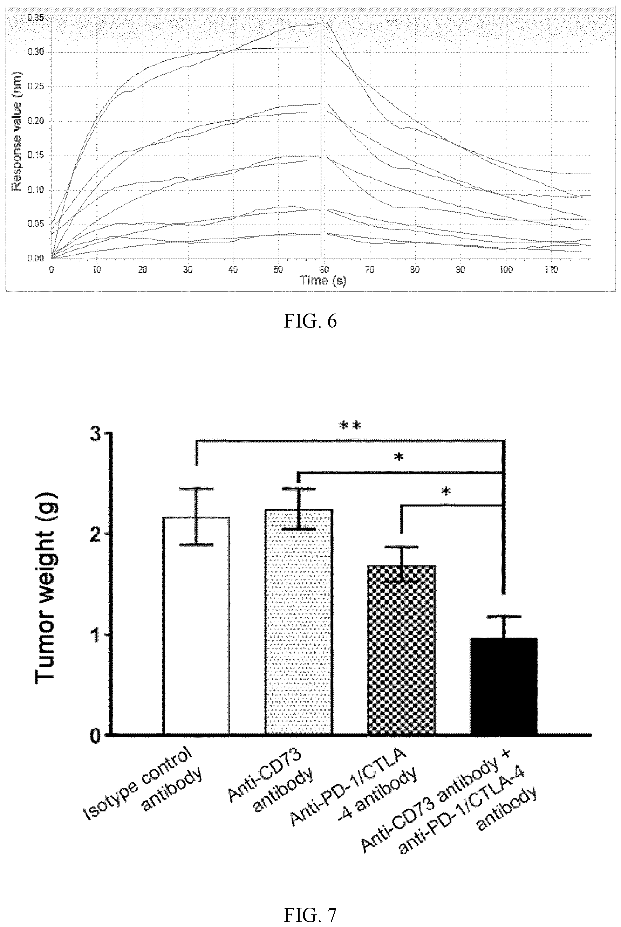

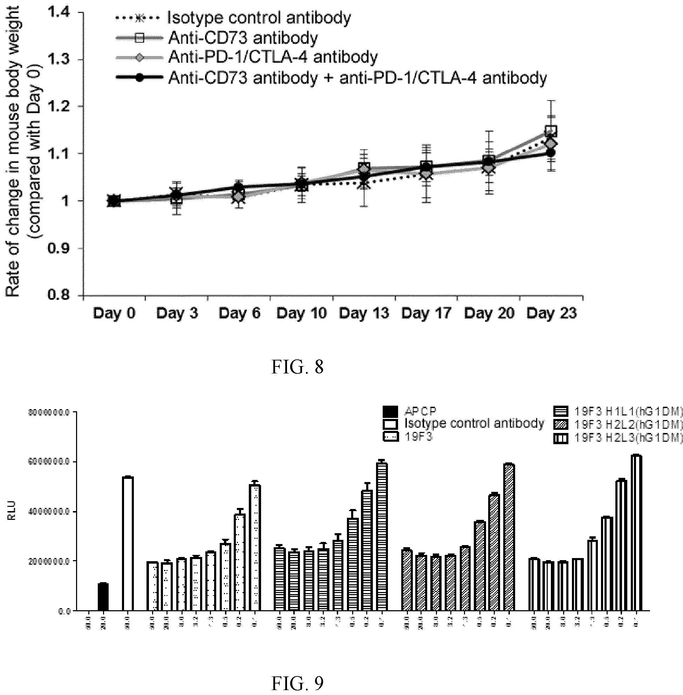

According to the results shown in FIG. 7, the combination of the anti-CD73 antibody and the anti-PD-1/CTLA-4 bispecific antibody showed a more excellent tumor-inhibiting effect than the anti-CD73 antibody alone and the anti-PD-1/CTLA-4 bispecific antibody alone, and had no significant effect on the body weight of the mice (FIG. 8).

**Table 3. Study design**

| **N (number of mice in each group)** | **Administration group** | **Dose of administration (mg/kg)** | **Starting time of administration** | **Frequency of actual administration** |
|---|---|---|---|---|
| 8 | Isotype control antibody | 30 | On Day 5 after inoculation, D0 | BIW × 3.5, 7 times in total, intraperitoneal injection |
| 8 | Anti-CD73 antibody | 30 | | |
| 8 | Anti-PD-1/CTLA-4 Antibody | 0.2 | | |
| 8 | Anti-PD-1/CTLA-4 Antibody | 0.2 | | |
| | Anti-CD73 antibody | 30 | | |

Mouse colon cancer cells MC38-hPDL1/hCD73 (provided by Gempharmatech Co., Ltd.) were thawed with the number of passages of Pn + 3. MC38-hPDL1/hCD73 cells in logarithmic growth phase were collected, and the culture solution was removed. The cells were washed twice with PBS and then inoculated into the right forelimb of C57BL/6-hPD1/hPDL1/hCD73 mice (provided by Gempharmatech Co., Ltd.) (99.1% MC38-hPDL1/hCD73 cell viability before tumor bearing and 96.4% cell viability after tumor bearing) in an amount of 2 × 10⁶/100 µL/mouse. On Day 5 after inoculation, when the mean tumor volume reached 86.02 mm³, 32 mice were randomized into 4 groups of 8 according to tumor volume. The day of grouping was defined as Day 0, and administration was started on Day 0 according to Table 3.

After cell inoculation, the effect of tumors on the normal behavior of the animals was routinely monitored weekly. The administration was performed on Day 0, Day 3, Day 7, Day 10, Day 14, Day 17, and Day 22. The tumor size was observed and the mice were weighed on Day 0, Day 3, Day 6, Day 10, Day 13, Day 17, Day 20, and Day 23. After the experiment was completed on Day 23, the tumor tissues from each group of animals were collected and weighed. The experimental results, such as the rate of change in mouse body weight, tumor weight, etc., were expressed as mean ± standard error (Mean ± SEM). The independent sample T-test was used to determine whether there were significant differences between different therapy groups and the control group, wherein *P* < 0.05 was considered to indicate a significant difference. Data were plotted using Graphpad or Excel.

### Example 6. Detection of Inhibition of Anti-CD73 Antibody on Enzyme Activity of CD73 Endogenously Expressed in Cells

The experimental procedures were as follows. MDA-MB-231 cells (from ATCC, HTB-26) in logarithmic phase in good condition were taken, resuspended in a serum-free RPMI-1640 culture solution, and then counted. The MDA-MB-231 cells were seeded into a 96-well plate at 3 × 10⁴ cells/100 µL/well. The antibody was diluted with the serum-free RPMI-1640 culture solution at an initial concentration of 200 µg/mL (serial 2.5-fold dilution). The antibody was added to the 96-well plate at 50 µL/well, and the plate was incubated at 37 °C for 1 h. After 1 h, 50 µL of RPMI-1640-diluted 600 µM AMP was added to each well. After 3 h, 25 µL of cell culture supernatant was taken and transferred to a new 96-well plate, and 25 µL of 100 µM ATP was added to each well. 50 µL of CTG (CellTiter-Glo^{®} One Solution Assay, promega, Cat No. G8461) color developing solution was added to each well for color development, and data were read by a multi-label microplate tester (PerkinElmer, Cat No. 2140-0020). The isotype control antibody and the CD73-specific inhibitor APCP were taken as negative and positive controls, respectively.

The experimental results were as follows: as shown in FIG. 9, 19F3, 19F3H2L3(hG1DM), 19F3H2L3(hG1DM) and 19F3H2L3(hG1DM) all showed dose-dependent inhibition of the activity of the endogenously-expressed CD73 enzyme catalyzing AMP to adenosine A in MDA-MB-231, thereby dose-dependently reducing the mean fluorescence intensity RLU produced.

The above experimental results showed that the added AMP could be catalyzed by CD73 enzyme expressed endogenously on the cell surface by MDA-MB-231 and then converted into adenosine under the condition of no CD73 antibody treatment, so that the inhibition of luciferase activity was relieved. However, after addition of the antibody, as CD73 was bound by the antibody, its enzymatic activity was reduced, so that AMP could not be converted into adenosine. It is suggested that the anti-CD73 antibody effectively inhibits the enzyme activity reaction of CD73 in a non-substrate competition mode and reduces the production of adenosine.

### SEQUENCE LISTING

The nucleotide sequence of the heavy chain variable region of 19F3: (SEQ ID NO: 1) 
GAGGTGCAGCTGCAGCAGTCCGGACCAGAGCTGGTGAAGCCTGGC
GCCTCCATGCGGATGTCTTGTAAGGCCTCTGGCTACAGCTTCACCGG
CTATACAATGAACTGGGTGAAGCAGTCTCACGGCAAGAATCTGGAGT
GGATCGGCCTGATCAACCCTTACAATGCCGGCACCAGCTATAACCAG
AAGTTTAAGGGCAAGGCCACCCTGACAGTGGACAAGAGCTCCTCTA
CCGCCTACATGGAGCTGCTGTCCCTGACATCTGAGGATAGCGCCGTG
TACTATTGCGCCCGGTCCGAGTACAGATATGGCGGCGACTACTTTGAT
TATTGGGGCCAGGGCACCACACTGACAGTGAGCTCC
The amino acid sequence of the heavy chain variable region of 19F3: (SEQ ID NO: 2) 
EVQLQQSGPELVKPGASMRMSCKASGYSFTGYTMNWVKQSHGKNLE
WIGLINPYNAGTSYNQKFKGKATLTVDKSSSTAYMELLSLTSEDSAVYY
CARSEYRYGGDYFDYWGQGTTLTVSS
The nucleotide sequence of the light chain variable region of 19F3: (SEQ ID NO: 3) 
GACATCGTGATGACCCAGTCTCCAAGCTCCCTGGCAATGAGCGTGGG
ACAGAAGGTGACAATGTCTTGTAAGTCTAGCCAGAGCCTGCTGAAC
TCCTCTAATCAGAAGAACTACCTGGCCTGGTATCAGCAGAAGCCAGG
CCAGTCTCCCAAGCTGCTGGTGTACTTTGCCAGCACCAGGGAGTCC
GGAGTGCCTGACAGATTCATCGGCTCCGGCTCTGGCACAGACTTCAC
CCTGACAATCAGCTCCGTGCAGGCAGAGGACCTGGCAGATTATTTCT
GCCAGCAGCACTACGACACCCCTTATACATTTGGCGGCGGCACCAA
GCTGGAGATCAAG
The amino acid sequence of the light chain variable region of 19F3: (SEQ ID NO: 4) 
DIVMTQSPSSLAMSVGQKVTMSCKSSQSLLNSSNQKNYLAWYQQKPG
QSPKLLVYFASTRESGVPDRFIGSGSGTDFTLTISSVQAEDLADYFCQQH
YDTPYTFGGGTKLEIK
The nucleotide sequence of the heavy chain variable region of 19F3H1L1(hG1DM): (SEQ ID NO: 5) 
CAGGTGCAGCTGCAGCAGTCTGGAGCAGAGGTGGTGAAGCCAGGA
GCCTCTATGAAGATGAGCTGTAAGGCCAGCGGCTACTCCTTCACCGG
CTATACAATGAACTGGGTGAAGCAGGCCCACGGCCAGAATCTGGAG
TGGATCGGCCTGATCAACCCTTACAATGCCGGCACCTCTTATAACCA
GAAGTTTCAGGGCAAGGCCACCCTGACAGTGGACAAGTCCACCTCT
ACAGCCTACATGGAGCTGAGCTCCCTGCGGAGCGAGGATACAGCCG
TGTACTATTGCGCCCGGTCCGAGTACAGATATGGCGGCGACTACTTTG
ATTATTGGGGCCAGGGCACCACACTGACCGTGTCTAGC
The amino acid sequence of the heavy chain variable region of 19F3H1L1(hG1DM): (SEQ ID NO: 6) 
QVQLQQSGAEVVKPGASMKMSCKASGYSFTGYTMNWVKQAHGQNL
EWIGLINPYNAGTSYNQKFQGKATLTVDKSTSTAYMELSSLRSEDTAVY
YCARSEYRYGGDYFDYWGQGTTLTVSS
The nucleotide sequence of the light chain variable region of 19F3H1L1(hG1DM): (SEQ ID NO: 7) 
GACATCGTGATGACCCAGTCCCCAAGCTCCCTGGCAATGTCTGTGGG
AGAGAGGGTGACAATGTCCTGTAAGTCTAGCCAGTCTCTGCTGAACT
CCTCTAATCAGAAGAACTACCTGGCCTGGTATCAGCAGAAGCCCGGC
CAGGCCCCTAAGCTGCTGGTGTACTTTGCCTCTACCAGGGAGAGCG
 
GAGTGCCAGACAGATTCTCTGGCAGCGGCTCCGGCACAGACTTCAC
CCTGACAATCAGCTCCGTGCAGGCAGAGGACCTGGCAGATTATTTCT
GCCAGCAGCACTACGATACCCCCTATACATTTGGCGGCGGCACCAAG
CTGGAGATCAAG
The amino acid sequence of the light chain variable region of 19F3H1L1(hG1DM): (SEQ ID NO: 8) 
DIVMTQSPSSLAMSVGERVTMSCKSSQSLLNSSNQKNYLAWYQQKPG
QAPKLLVYFASTRESGVPDRFSGSGSGTDFTLTISSVQAEDLADYFCQQ
HYDTPYTFGGGTKLEIK
The nucleotide sequences of the heavy chain variable regions of 19F3H2L2(hG1DM) and 19F3H2L3(hG1DM): (SEQ ID NO: 9) 
CAGGTGCAGCTGGTGCAGTCTGGAGCAGAGGTGGTGAAGCCAGGA
GCCTCTGTGAAGGTGAGCTGTAAGGCCAGCGGCTACTCCTTCACCG
GCTATACAATGAACTGGGTGAGGCAGGCACCAGGACAGAATCTGGA
GTGGATCGGCCTGATCAACCCTTACAATGCCGGCACCTCTTATAACC
AGAAGTTTCAGGGCAAGGTGACCCTGACAGTGGACAAGTCCACCTC
TACAGCCTACATGGAGCTGAGCTCCCTGCGGAGCGAGGATACAGCC
GTGTACTATTGCGCCCGGTCCGAGTACAGATATGGCGGCGACTACTTT
GATTATTGGGGCCAGGGCACCACACTGACCGTGTCTAGC
The amino acid sequences of the heavy chain variable regions of 19F3H2L2(hG1DM) and 19F3H2L3(hG1DM): (SEQ ID NO: 10) 
QVQLVQSGAEVVKPGASVKVSCKASGYSFTGYTMNWVRQAPGQNLE
WIGLINPYNAGTSYNQKFQGKVTLTVDKSTSTAYMELSSLRSEDTAVYY
CARSEYRYGGDYFDYWGQGTTLTVSS
The nucleotide sequence of the light chain variable region of 19F3H2L2(hG1DM): (SEQ ID NO: 11) 
GACATCGTGATGACCCAGTCCCCAAGCTCCCTGGCCGTGTCTGTGGG
AGAGCGGGTGACAATCTCCTGTAAGTCTAGCCAGTCTCTGCTGAACT
CCTCTAATCAGAAGAACTACCTGGCCTGGTATCAGCAGAAGCCCGGC
CAGGCCCCTAAGCTGCTGATCTACTTCGCCTCTACCAGGGAGAGCGG
AGTGCCAGACAGATTCTCTGGCAGCGGCTCCGGCACAGACTTCACC
CTGACAATCAGCTCCGTGCAGGCAGAGGACGTGGCAGATTACTATTG
CCAGCAGCACTACGATACCCCCTATACATTTGGCGGCGGCACCAAGC
TGGAGATCAAG
The amino acid sequence of the light chain variable region of 19F3H2L2(hG1DM): (SEQ ID NO: 12) 
DIVMTQSPSSLAVSVGERVTISCKSSQSLLNSSNQKNYLAWYQQKPGQA
PKLLIYFASTRESGVPDRFSGSGSGTDFTLTISSVQAEDVADYYCQQHY
DTPYTFGGGTKLEIK
The nucleotide sequence of the light chain variable region of 19F3H2L3(hG1DM): (SEQ ID NO: 13) 
GACATCGTGATGACCCAGTCCCCAAGCTCCCTGGCCGTGTCTGTGGG
AGAGCGGGTGACAATCTCCTGTAAGTCTAGCCAGTCTCTGCTGAACT
CCTCTAATCAGAAGAACTACCTGGCCTGGTATCAGCAGAAGCCCGGC
CAGGCCCCTAAGCTGCTGATCTACTTCGCCTCTACCAGGGAGAGCGG
AGTGCCAGACAGATTCTCTGGCAGCGGCTCCGGCACAGACTTCACC
CTGACAATCAGCTCCCTGCAGGCAGAGGACGTGGCCGTGTACTATTG
CCAGCAGCACTACGATACCCCCTATACATTTGGCGGCGGCACCAAGC
TGGAGATCAAG
The amino acid sequence of the light chain variable region of 19F3H2L3(hG1DM): (SEQ ID NO: 14) 
DIVMTQSPSSLAVSVGERVTISCKSSQSLLNSSNQKNYLAWYQQKPGQA
PKLLIYFASTRESGVPDRFSGSGSGTDFTLTISSLQAEDVAVYYCQQHYD
TPYTFGGGTKLEIK
The CDRs of 19F3 and 19F3H1L1(hG1DM), 19F3H2L2(hG1DM), and 19F3H2L3(hGlDM)
   HCDR1: GYSFTGYT (SEQ ID NO: 15)
   HCDR2: INPYNAGT (SEQ ID NO: 16)
   HCDR3: ARSEYRYGGDYFDY (SEQ ID NO: 17)
   LCDR1: QSLLNSSNQKNY (SEQ ID NO: 18)
   LCDR2: FAS (SEQ ID NO: 19)
   LCDR3: QQHYDTPYT (SEQ ID NO: 20)
The sequences of the heavy chain constant regions (330 aa, mutation sites underlined) of 19F3H1L1(hG1DM), 19F3H2L2(hG1DM) and 19F3H2L3 (hG1DM): 
ASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKV
EPKSCDKTHTCPPCPAPEAAGGPSVFLFPPKPKDTLMISRTPEVTCVVV
DVSHEDPEVKFNWYVDGVEVHNAKTKPREEQYNSTYRVVSVLTVLHQ
DWLNGKEYKCKVSNKALPAPIEKTISKAKGQPREPQVYTLPPSRDELTK
NQVSLTCLVKGFYPSDIAVEWESNGQPENNYKTTPPVLDSDGSFFLYSK
LTVDKSRWQQGNVFSCS VMHEALHNHYTQKSLSLSPGK (SEQ ID NO:
21)
The sequences of the light chain constant regions (107 aa) of 19F3H1L1(hG1DM), 19F3H2L2(hG1DM) and 19F3H2L3(hG1DM): 


 (SEQ ID NO: 22)
The amino acid sequences of the 9 CDRs associated with the heavy chain variable region of BiAb004(hG1TM) are as follows:
   HCDR1: GFAFSSYD (SEQ ID NO: 23)
   HCDR2: ISGGGRYT (SEQ ID NO:24)
   HCDR3: ANRYGEAWFAY (SEQ ID NO: 25)
   HCDR4: GYSFTGYT (SEQ ID NO: 26)
   HCDR5: INPYNNIT (SEQ ID NO: 27)
   HCDR6: ARLDYRSY (SEQ ID NO: 28)
   HCDR7: TGAVTTSNF (SEQ ID NO: 29)
   HCDR8: GTN (SEQ ID NO: 30)
   HCDR9: ALWYSNHWV (SEQ ID NO:31)
The amino acid sequences of the 3 CDRs associated with the light chain variable region of BiAb004(hG1TM) are as follows:
   LCDR1: QDINTY (SEQ ID NO: 32)
   LCDR2: RAN (SEQ ID NO: 33)
   LCDR3: LQYDEFPLT (SEQ ID NO: 34)
The amino acid sequence of the heavy chain (713 aa, mutation sites underlined) of BiAb004(hG1TM) 
EVQLVESGGGLVQPGGSLRLSCAASGFAFSSYDMSWVRQAPGKGLDW
VATISGGGRYTYYPDSVKGRFTISRDNSKNNLYLQMNSLRAEDTALYYC
ANRYGEAWFAYWGQGTLVTVSSASTKGPSVFPLAPSSKSTSGGTAALG
CLVKDYFPEPVTVSWNSGALTSGVHTFPAVLQSSGLYSLSSVVTVPSSSL
GTQTYICNVNHKPSNTKVDKKVEPKSCDKTHTCPPCPAPEAAGAPSVF
LFPPKPKDTLMISRTPEVTCVVVDVSHEDPEVKFNWYVDGVEVHNAK
 
TKPREEQYNSTYRVVSVLTVLHQDWLNGKEYKCKVSNKALPAPIEKTI
SKAKGQPREPQVYTLPPSRDELTKNQVSLTCLVKGFYPSDIAVEWESNG
QPENNYKTTPPVLDSDGSFFLYSKLTVDKSRWQQGNVFSCSVMHEALH
NHYTQKSLSLSPGKGGGGSGGGGSGGGGSGGGGSQVQLVESGAEVKK
PGASVKVSCKASGYSFTGYTMNWVRQAPGQCLEWIGLINPYNNITNYA
QKFQGRVTFTVDTSISTAYMELSRLRSDDTGVYFCARLDYRSYWGQGT
LVTVSAGGGGSGGGGSGGGGSGGGGSQAVVTQEPSLTVSPGGTVTLTC
GSSTGAVTTSNFPNWVQQKPGQAPRSLIGGTNNKASWTPARFSGSLLG
GKAALTISGAQPEDEAEYYCALWYSNHWVFGCGTKLTVLR (SEQ ID
NO: 35)
The amino acid sequence of the light chain (214 aa) of BiAb004(hG 1 TM)

DIQMTQSPSSMSASVGDRVTFTCRASQDINTYLSWFQQKPGKSPKTLIY
RANRLVSGVPSRFSGSGSGQDYTLTISSLQPEDMATYYCLQYDEFPLTF
GAGTKLELKRTVAAPSVFIFPPSDEQLKSGTASVVCLLNNFYPREAKVQ
WKVDNALQSGNSQESVTEQDSKDSTYSLSSTLTLSKADYEKHKVYACE
VTHQGLSSPVTKSFNRGEC (SEQ ID NO: 36)

## Claims

1. An anti-CD73 (for example, human CD73) antibody or an antigen-binding fragment thereof, wherein according to an IMGT numbering system, the anti-CD73 antibody comprises:
HCDR1, HCDR2 and HCDR3 of a heavy chain variable region set forth in SEQ ID NO: 2, SEQ ID NO: 6 or SEQ ID NO: 10; and LCDR1, LCDR2 and LCDR3 of a light chain variable region set forth in SEQ ID NO: 4, SEQ ID NO: 8, SEQ ID NO: 12 or SEQ ID NO: 14;
preferably, according to the IMGT numbering system, the anti-CD73 antibody comprises:
HCDR1 comprising or consisting of an amino acid sequence set forth in SEQ ID NO: 15, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
HCDR2 comprising or consisting of an amino acid sequence set forth in SEQ ID NO: 16, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
HCDR3 comprising or consisting of an amino acid sequence set forth in SEQ ID NO: 17, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
LCDR1 comprising or consisting of an amino acid sequence set forth in SEQ ID NO: 18, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence,
LCDR2 comprising or consisting of an amino acid sequence set forth in SEQ ID NO: 19, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence, and
LCDR3 comprising or consisting of an amino acid sequence set forth in SEQ ID NO: 20, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2 or 3) conservative amino acid mutations (preferably substitutions, insertions or deletions) compared with the sequence.

2. The anti-CD73 antibody or the antigen-binding fragment thereof according to claim 1, wherein a heavy chain variable region of the antibody comprises or consists of the following sequences:
SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to SEQ ID NO: 2, SEQ ID NO: 6 or SEQ ID NO: 10, or an amino acid sequence having one or more (preferably 1, 2, 3, 4, 5, 6, 7, 8, 9 or 10) conservative amino acid mutations (preferably substitutions, insertions or deletions) compared with the amino acid sequence set forth in SEQ ID NO: 2, SEQ ID NO: 6 or SEQ ID NO: 10; and
a light chain variable region of the antibody comprises or consists of the following sequences:
SEQ ID NO: 4, SEQ ID NO: 8, SEQ ID NO: 12 or SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to SEQ ID NO: 4, SEQ ID NO: 8, SEQ ID NO: 12 or SEQ ID NO: 14, or an amino acid sequence having one or more (preferably 1, 2, 3, 4, 5, 6, 7, 8, 9 or 10) conservative amino acid mutations (preferably substitutions, insertions or deletions) compared with the amino acid sequence set forth in SEQ ID NO: 4, SEQ ID NO: 8, SEQ ID NO: 12 or SEQ ID NO: 14.

3. The anti-CD73 antibody or the antigen-binding fragment thereof according to claim 1 or 2, wherein
the heavy chain variable region of the antibody comprises an amino acid sequence set forth in SEQ ID NO: 2, and the light chain variable region of the antibody comprises an amino acid sequence set forth in SEQ ID NO: 4;
the heavy chain variable region of the antibody comprises an amino acid sequence set forth in SEQ ID NO: 6, and the light chain variable region of the antibody comprises an amino acid sequence set forth in SEQ ID NO: 8;
the heavy chain variable region of the antibody comprises an amino acid sequence set forth in SEQ ID NO: 10, and the light chain variable region of the antibody comprises an amino acid sequence set forth in SEQ ID NO: 12; or the heavy chain variable region of the antibody comprises an amino acid sequence set forth in SEQ ID NO: 10, and the light chain variable region of the antibody comprises an amino acid sequence set forth in SEQ ID NO: 14.

4. The anti-CD73 antibody or the antigen-binding fragment thereof according to claim 1 or 2, wherein a heavy chain constant region of the antibody is an Ig gamma-1 chain C region, ACCESSION: P01857; a light chain constant region is an Ig kappa chain C region, ACCESSION: P01834.

5. The anti-CD73 antibody or the antigen-binding fragment thereof according to claim 1 or 2, wherein the antibody is a monoclonal antibody, a humanized antibody, a chimeric antibody or a multispecific antibody (e.g., a bispecific antibody).

6. The anti-CD73 antibody or the antigen-binding fragment thereof according to claim 1 or 2, wherein the antigen-binding fragment is selected from Fab, Fab', F(ab')₂, Fd, Fv, dAb, Fab/c, a complementarity determining region fragment, a single chain antibody (e.g., scFv), a humanized antibody, a chimeric antibody and a bispecific antibody.

7. An isolated polypeptide, selected from the group consisting of:
(1) an isolated polypeptide, comprising sequences set forth in SEQ ID NO: 15, SEQ ID NO: 16 and SEQ ID NO: 17, wherein the polypeptide, as part of an anti-CD73 antibody, specifically binds to CD73, the antibody further comprising sequences set forth in SEQ ID NO: 18, SEQ ID NO: 19 and SEQ ID NO: 20;
(2) an isolated polypeptide, comprising sequences set forth in SEQ ID NO: 18, SEQ ID NO: 19 and SEQ ID NO: 20, wherein the polypeptide, as part of an anti-CD73 antibody, specifically binds to CD73, the antibody further comprising sequences set forth in SEQ ID NO: 15, SEQ ID NO: 16 and SEQ ID NO: 17;
(3) an isolated polypeptide, comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence;
(4) an isolated polypeptide, comprising a sequence set forth in SEQ ID NO: 4, SEQ ID NO: 8 or SEQ ID NO: 12, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 2, SEQ ID NO: 6 or SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence;
(5) an isolated polypeptide, comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence; and
(6) an isolated polypeptide, comprising a sequence set forth in SEQ ID NO: 14, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence, wherein the polypeptide, as part of an anti-CD73 antibody, specifically binds to CD73, the antibody further correspondingly comprising a sequence set forth in SEQ ID NO: 10, a sequence having at least 80%, 81%, 82%, 83%, 84%, 85%, 86%, 87%, 88%, 89% or 90%, preferably at least 91%, 92%, 93%, 94%, 95%, 96%, 97%, 98% or 99% sequence identity to the sequence, or an amino acid sequence having one or more (preferably 1, 2, 3, 4, 5, 6, 7, 8, 9 or 10) conservative amino acid mutations (preferably substitutions, insertions or deletions) compared with the sequence.

8. A nucleic acid molecule, encoding the antibody or the antigen-binding fragment thereof according to any one of claims 1 to 6 or the isolated polypeptide according to claim 7.

9. A vector, comprising the nucleic acid molecule according to claim 8.

10. A host cell, comprising the nucleic acid molecule according to claim 8 or the vector according to claim 9.

11. A conjugate, comprising the antibody or the antigen-binding fragment thereof according to any one of claims 1 to 6 and a conjugated moiety, wherein the conjugated moiety is a purification tag (e.g., a His tag) or a detectable label; preferably, the conjugated moiety is a radioisotope, a fluorescent substance, a chemiluminescent substance, a colored substance, polyethylene glycol or an enzyme.

12. A fusion protein or a multispecific antibody (preferably a bispecific antibody), comprising the antibody or the antigen-binding fragment thereof according to any one of claims 1 to 6.

13. A kit, comprising the antibody or the antigen-binding fragment thereof according to any one of claims 1 to 6, the conjugate according to claim 11, or the fusion protein or the multispecific antibody according to claim 12, wherein preferably, the kit further comprises a second antibody specifically recognizing the antibody; optionally, the second antibody further comprises a detectable label, such as a radioisotope, a fluorescent substance, a chemiluminescent substance, a colored substance or an enzyme; preferably, the kit is used for detecting the presence or level of CD73 in a sample.

14. A pharmaceutical composition, comprising the antibody or the antigen-binding fragment thereof according to any one of claims 1 to 6, the conjugate according to claim 11, or the fusion protein or the multispecific antibody according to claim 12, wherein optionally, the pharmaceutical composition further comprises a pharmaceutically acceptable carrier and/or excipient; preferably, the pharmaceutical composition is in a form suitable for administration by subcutaneous injection, intradermal injection, intravenous injection, intramuscular injection or intralesional injection.

15. Use of the antibody or the antigen-binding fragment thereof according to any one of claims 1 to 6, the conjugate according to claim 11, or the fusion protein or the multispecific antibody according to claim 12 in preparing a medicament for treating and/or preventing a tumor (such as a solid tumor, preferably non-small cell lung cancer, prostate cancer (including metastatic castration-resistant prostate cancer (mCRPC)), triple-negative breast cancer, ovarian cancer, colorectal cancer (including microsatellite stability (MSS) and mismatch repair dysfunction/microsatellite high instability (dMMR/MSI-high) type), gastric cancer (including microsatellite stability (MSS) and mismatch repair dysfunction/microsatellite high instability (dMMR/MSI-high) type), melanoma, head and neck cancer, renal cell carcinoma or pancreatic ductal adenocarcinoma), or in preparing a medicament for diagnosing a tumor.

16. A hybridoma cell line, selected from:
LT014 deposited at China Center for Type Culture Collection (CCTCC) under CCTCC NO. C2018137.

17. A kit, comprising (1) the antibody or the antigen-binding fragment thereof according to any one of claims 1 to 6, the conjugate according to claim 11, or the fusion protein or the multispecific antibody according to claim 12, and (2) an anti-PD-1/CTLA-4 bispecific antibody, and optionally, instructions for use; preferably, the anti-PD-1/CTLA-4 bispecific antibody comprises a heavy chain amino acid sequence set forth in SEQ ID NO: 35 and a light chain amino acid sequence set forth in SEQ ID NO: 36.

18. A method for treating and/or preventing a tumor, comprising administering to a patient a therapeutically effective amount of drug A and a therapeutically effective amount of drug B, wherein drug A comprises the antibody or the antigen-binding fragment thereof according to any one of claims 1 to 6, the conjugate according to claim 11, or the fusion protein or the multispecific antibody according to claim 12, and drug B comprises an anti-PD-1/CTLA-4 bispecific antibody; preferably, drug A and drug B are administered either simultaneously or sequentially, wherein the sequential administration is that drug A is administrated firstly or drug B is administrated firstly; preferably, the anti-PD-1/CTLA-4 bispecific antibody comprises a heavy chain amino acid sequence set forth in SEQ ID NO: 35 and a light chain amino acid sequence set forth in SEQ ID NO: 36.
